(19) Europäisches Patentamt
European Patent Office
Office européen des brevets

(11) Numéro de publication : **0 022 737**
**B1**

(12) # FASCICULE DE BREVET EUROPÉEN

(45) Date de publication du fascicule du brevet : **09.06.82**

(21) Numéro de dépôt : **80450003.1**

(22) Date de dépôt : **07.07.80**

(51) Int. Cl.³ : **C 07 D405/12**, C 07 D407/12, C 07 D409/12, A 61 K 31/44, A 61 K 31/38, A 61 K 31/335

(54) **Imines dérivées de l'amino-5 benzodioxole-1,3 utiles comme médicaments et leur préparation.**

(30) Priorité : **16.07.79 FR 7918357**
**21.04.80 FR 8008858**

(43) Date de publication de la demande : **21.01.81 (Bulletin 81/03)**

(45) Mention de la délivrance du brevet : **09.06.82 Bulletin 82/23**

(84) Etats contractants désignés : **BE CH DE GB IT LI LU NL**

(56) Documents cités :
**FR A 2 316 938**
**CHEMICAL ABSTRACTS, vol. 87, page 113, réf. 48917c Columbus, Ohio, US**

(73) Titulaire : **LABORATOIRES SARGET S.A.**
**Avenue du Président JF Kennedy**
**F-33701 Merignac (FR)**

(72) Inventeur : **Creuzet, Marie-Hélène**
**Résidence Jardin de Gambetta T3**
**F-33000 Bordeaux (FR)**
Inventeur : **Feniou, Claude**
**5, Rue du Général Guillomat**
**F-33600 Pessac (FR)**
Inventeur : **Prat, Gisèle**
**Hameau de Noailles Villa 18**
**F-33400 Talence (FR)**
Inventeur : **Pontagnier, Henri**
**21, rue Edouard Vaillant**
**F-33600 Pessac (FR)**

(74) Mandataire : **Tajan, Marie-Thérèse**
**LABORATOIRES SARGET Avenue du Président JF Kennedy**
**F-33701 Merignac (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 022 737 B1

Imprimerie Jouve, 18, rue St-Denis, 75001 Paris, France

# Imines dérivées de l'amino-5 benzodioxole-1,3 utiles comme médicaments et leur préparation

La présente invention concerne de nouvelles imines dérivées de l'amino-5 benzodioxole-1,3 utiles comme médicaments.

On connaît déjà des dérivés de l'amino-5 benzodioxole-1,3 ; en particulier la demande de brevet français n° 2 316 938 concerne des amino-3 3H-isobenzofurannones N-substituées contenant le reste amino-5 benzodioxole-1,3. Nous décrivons maintenant de nouveaux dérivés de formule générale

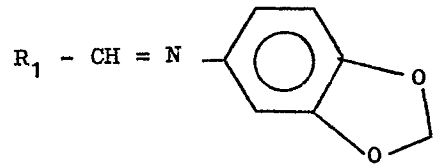

avec $R_1$ = reste hétérocyclique choisi dans le groupe

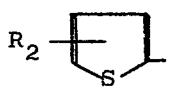 , 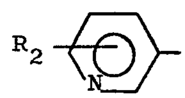 , 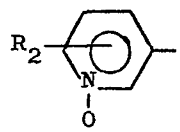 , 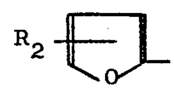

et $R_2$ = H ou un ou plusieurs substituants choisis dans le groupe $CH_3$, halogène, nitro.

Ces produits sont utiles en thérapeutique du fait de leurs activités analgésiques et anti-inflammatoires et présentent l'avantage d'être pratiquement dénués d'activité ulcérigène contrairement aux anti-inflammatoires classiques.

Ces produits sont également utiles dans le traitement oral du diabète. Celui-ci utilise des médicaments appartenant à deux familles chimiques, les sulfamides et les biguanides. Les sulfamides hypoglycémiants agissent par l'intermédiaire du pancréas en stimulant les cellules β des îlots de Langerhans. Les biguanides agissent par un mécanisme extrapancréatique en augmentant l'utilisation du glucose, en inhibant la néoglucogenèse hépatique et en diminuant l'absorption digestive du glucose. Ils présentent toutefois l'inconvénient d'entraîner des acidoses lactiques rares mais souvent mortelles. Cela a conduit en France à l'abandon de la commercialisation de la phenformine, seule celle de la metformine étant maintenue.

Les produits de la présente invention sont obtenus de façon générale par une réaction mettant en jeu l'amino-5 benzodioxole-1,3 et l'aldéhyde $R_1CHO$ avec $R_1$ ayant la signification indiquée ci-dessus, dans un solvant approprié tel que le benzène, l'eau produite au cours de la réaction étant éliminée au fur et à mesure de sa formation.

La mise en œuvre de l'invention sera expliquée de façon plus précise dans les exemples suivants.

## Exemple 1

### Synthèse du N-thiénylidèneamino-5 benzodioxole-1,3

Le mélange de 10 g d'amino-5 benzodioxole-1,3, 11 g de thiophènecarboxaldéhyde-2 et 70 $cm^3$ de benzène est chauffé à 120 °C sous agitation. L'eau formée au cours de la réaction est éliminée à l'aide d'un piège de Dean-Stark. Le benzène est éliminé par évaporation et l'imine distillée.

Caractéristiques

Masse moléculaire : 231 Formule brute : $C_{12}H_9NO_2S$
Solide jaune, insoluble dans l'eau
F = 62 °C
Spectre de RMN dans $CDCl_3$ étalon interne TMS

| | | | |
|---|---|---|---|
| 5,9 ppm | singulet | 2 protons | 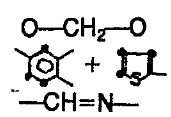 |
| 6,6-7,5 ppm | massif complexe | 6 protons | |
| 8,5 ppm | singulet | 1 proton | |

## Exemple 2

### Synthèse du N-(méthyl-5 thiénylidène-2) amino-5 benzodioxole-1,3

Le mélange de 10 g d'amino-5 benzodioxole-1,3, 12 g de méthyl-5 thiophène carboxaldéhyde-2 et 70 $cm^3$ de benzène est chauffé à 120 °C sous agitation. L'eau formée au cours de la réaction est éliminée à l'aide d'un piège de Dean-Stark. Le benzène est éliminé par évaporation et l'imine purifiée par distillation. Rendement 90 %.

Caractéristiques

Masse moléculaire : 245 Formule brute : $C_{13}H_{11}NO_2S$
Poudre jaune, insoluble dans l'eau
F = 73 °C
Spectre RMN dans $CDCl_3$ étalon interne TMS

| | | | |
|---|---|---|---|
| 2,5 ppm | singulet | 3 protons | $CH_3$ |
| 5,8 ppm | singulet | 2 protons | $—O—CH_2—O—$ |
| 6,5-7,2 ppm | massif complexe | 5 protons | 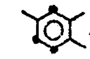 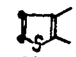 |
| 8,3 ppm | singulet | 1 proton | —CH=N— |

## Exemple 3

Synthèse du N-(pyridylidène-3) amino-5 benzodioxole-1,3

Ce produit est synthétisé comme les deux précédents à partir d'un mélange de 10 g d'amino-5 benzodioxole-1,3, 10 g de pyridine carboxaldéhyde-3 et 70 $cm^3$ de benzène.

Caractéristiques

Masse moléculaire : 226,2 Formule brute $C_{13}H_{10}N_2O_2$
Solide jaune, insoluble dans l'eau
F = 91 °C
Spectre de RMN dans $CDCl_3$ étalon interne TMS

| | | | |
|---|---|---|---|
| 5,9 ppm | singulet | 2 protons | $O—CH_2—O$ |
| 6,7-9 ppm | massif complexe | 8 protons | 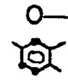 + 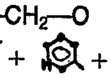 + —CH=N— (singulet à 8,4 ppm) |

## Exemple 4

Synthèse du N-(méthyl-3 thiénylidène-2) amino-5 benzodioxole-1,3

Cette imine est synthétisée comme décrit dans l'exemple 2. Après distillation elle est dissoute dans l'éther éthylique auquel de l'éther de pétrole est rajouté pour la faire cristalliser. Elle est ensuite filtrée et séchée sous vide à 50 °C. Rendement 85 %.

Caractéristiques

Masse moléculaire : 245 Formule brute $C_{13}H_{11}NO_2S$
Poudre jaune, insoluble dans l'eau
F = 69 °C
Spectre de RMN dans $CDCl_3$ étalon interne TMS

| | | | |
|---|---|---|---|
| 2,4 ppm | singulet | 3 protons | $CH_3$ |
| 5,9 ppm | singulet | 2 protons | $—O—CH_2—O—$ |
| 6,6-7,4 ppm | massif complexe | 5 protons | 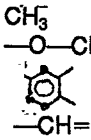 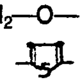 |
| 8,5 ppm | singulet | 1 proton | —CH=N— |

## Résultats pharmacologiques

Les pourcentages de mortalité induits par les produits de la présente invention administrés par voie orale chez la souris Swiss sont représentés dans le tableau 1. Les animaux exempts d'organismes pathogènes spécifiques sont stabulés en salle climatisée 24 à 48 h avant le début de l'expérimentation. Ils sont répartis en lots de 5 mâles et 5 femelles. Chez les animaux à jeun depuis 24 h, les substances sont

administrées par voie intragastrique en suspension dans un julep gommeux à 6 % sous un volume équivalent à 0,1 ml par 10 g de poids d'animal. Après traitement les animaux sont observés pendant une heure puis toutes les heures pendant la première journée ; ils restent en observation pendant deux semaines avant d'être sacrifiés et autopsiés.

Tableau 1

Toxicité chez la souris

| Produit de l'exemple | % de mortalité en fonction de la dose exprimée en mg/kg | | | | | | | |
|---|---|---|---|---|---|---|---|---|
| | 500 | 750 | 1000 | 1250 | 1500 | 1750 | 2000 | 3000 |
| 1 | | | 0 | | 20 | | 60 | 70 |
| 2 | | 10 | 20 | 0 | 60 | | | |
| 3 | 0 | | 10 | | | | 100 | |
| 4 | | 10 | 10 | | 60 | 20 | 20 | |

La toxicité des produits de l'exemple 2 et 3 a été déterminée chez le rat Wistar. Le protocole suivi est le même que celui décrit pour la souris à la seule exception près que le produit est administré sous un volume équivalent à 0,5 ml pour 100 g de poids de rat. Le produit de l'exemple 2 ne détermine aucune mortalité à 1 500 mg/kg. Le produit de l'exemple 3 n'entraîne aucune mortalité à 2 000 mg/kg et 20 % de mortalité à 3 000 mg/kg.

L'activité analgésique a été déterminée chez la souris mâle Swiss en utilisant comme agent nociceptif la phénylbenzoquinone (PBQ) (variante de la méthode de Siegmund & coll. Proc. Soc. Exp. Biol. Med., 1975, 95, 729-31). Vingt-cinq minutes après avoir administré par voie orale les produits à éprouver en suspension dans un julep gommeux à 6 %, on injecte la solution de PBQ puis on observe les souris au bout de 5 mn pendant les 5 mn suivantes. Les résultats sont rassemblés dans le tableau 2. La DE 50 est déterminée par la méthode de Lichfield Wilcoxon.

Tableau 2

| Produit de l'exemple | % d'activité analgésique en fonction de la dose exprimée en mg/kg | | | | | | | | |
|---|---|---|---|---|---|---|---|---|---|
| | 50 | 75 | 100 | 125 | 150 | 200 | 250 | 300 | DE 50 |
| 1 | | | 34 | 43 | 69 | 73 | | | 130 (99 - 171) |
| 2 | | | 19 | | | 49 | 51 | 40 | |
| 3 | 23 | | 44 | | | 65 | | 92 | 110 (67 - 179) |
| 4 | | 21 | 40 | | 37 | 62 | | 67 | 170 (107 - 270 |

Le tableau 3 donne les pourcentages d'activité inhibitrice de l'œdème à la carragénine. L'inflammation est induite chez le rat mâle Wistar par injection de 0,1 ml d'une suspension à 0,5 % de carragénine dans du sérum physiologique, dans le faisceau musculaire de la région métatarsienne de la patte postérieure de l'animal. La substance à éprouver est administrée par voie orale en même temps que la carragénine. L'œdème est mesuré par pléthysmométrie 3 h après administration du carragénine.

Tableau 3

| Traitement | Moment d'administration du produit | % d'activité inhibitrice de l'oedème à la carragénine en fonction de la dose exprimée en mg/kg | | | | | | | | | | |
|---|---|---|---|---|---|---|---|---|---|---|---|---|
| | | 25 | 50 | 75 | 100 | 125 | 150 | 200 | 225 | 300 | 400 | 450 |
| Produit de l'exemple 1 | $T_0$ | 19 | 41 | | 52 | | | 56 | | | 58 | |
| Produit de l'exemple 2 | $T_0$ | | | | 30 | | 64 | 33 | | 59 | | |
| | $T_0$ - 30 | | | 21 | | | 47 | | | 61 | | |
| | $T_0$ - 60 | | | 15 | | | 26 | | 49 | 66 | | |
| Produit de l'exemple 3 | $T_0$ | | | | 57<br>29 | | | 28<br>36 | | | 55<br>39 | |
| | $T_0$ - 30 | 34 | 41<br>38 | 44 | 50<br>41 | | 53 | 66 | | | 67<br>72 | |
| | $T_0$ - 60 | 25 | 42 | 40 | 39 | | | 58 | | | 69 | |
| Produit de l'exemple 4 | $T_0$ | | | 35 | | | 35 | | | 68 | | |
| Aspirine | $T_0$ | | | 40 | | | 46 | | | 68 | | 64 |
| Acide niflumique | $T_0$ | | | 37 | 27 | 40 | 37<br>59 | | | | | |

Le tableau 4 indique les pourcentages d'activité inhibitrice de l'œdème à la sérotonine. Chez le rat Wistar l'inflammation est induite par l'injection intrapédieuse de 0,1 ml d'une solution à 0,02 % de sérotonine dans du sérum physiologique. La substance à éprouver est administrée par voie orale 30 mn avant la sérotonine et l'œdème est mesuré par pléthysmométrie 1 h après son déclenchement.

Tableau 4

| Produit de l'exemple | % d'activité inhibitrice en fonction de la dose exprimée en mg/kg | | |
|---|---|---|---|
| | 100 | 200 | 400 |
| 2 | 39 | 29 | 37 |
| 3 | 27 | 21 | 26 |

Le tableau 5 représente l'activité vis-à-vis des granulomes à la carragénine. Des rats mâles Wistar de 130 à 150 g reçoivent dans la région médiane de la peau du dos une injection sous cutanée de 0,5 ml d'une suspension aqueuse de carragénine. Au bout de 24 h les rats sont sacrifiés et les granulomes prélevés et pesés avant et après un séchage de 24 h à 50 °C. La différence des poids des granulomes humides-granulomes secs représente l'exsudat.

Tableau 5

| Produit de l'exemple | Dose en mg/kg administrée 2 fois | Poids du granulome humide | | Poids du granulome sec | | Exsudat | |
|---|---|---|---|---|---|---|---|
| | | % activité | % animaux protégés à 30% | % activité | % animaux protégés à 30% | % activité | % animaux protégés à 30% |
| 1 | 200 | 6 | 12,5 | 1 | 12,5 | 6 | 12,5 |
| 2 | 100 | 32<br>15 | 62<br>25 | 34<br>14 | 62<br>25 | 31 | 62 |
| | 150 | 5<br>22 | 25<br>14 | 1<br>24 | 25<br>14 | 6<br>22 | 25<br>14 |
| | 200 | 39<br>24 | 75<br>50 | 41<br>22 | 75<br>50 | 39 | 75 |
| | 300 | 18 | 25 | 9 | 25 | 18 | 2 |
| 3 | 100 | 15<br>25 | 25<br>37 | 12<br>22 | 12<br>25 | 15<br>25 | 25<br>37 |
| | 200 | 27<br>33<br>34 | 37<br>75<br>71 | 27<br>32<br>33 | 37<br>75<br>71 | 27<br>34<br>38 | 37<br>75<br>71 |
| | 250 | 36 | 75 | 33 | 62 | 36 | 75 |
| | 300 | 35<br>43 | 87<br>75 | 37<br>41 | 62<br>75 | 35<br>43 | 87<br>75 |
| 4 | 100 | 20,5 | 38 | 16 | 31 | 21 | 38 |
| | 200 | 34 | 71 | 31 | 64 | 34,5 | 71 |
| | 300 | 44,5 | 75 | 42,5 | 67 | 44,5 | 75 |
| Acide niflumique | 25 | 42,5 | 100 | 41,5 | 87 | 42,5 | 100 |

Ces résultats ont permis de calculer pour les produits des exemples 2 et 3 les DA 50 (doses protégeant 50 % des animaux à 30 % vis-à-vis des poids des granulomes humides). Celles-ci sont respectivement de 175 (142-215) et 175 (150-205) mg/kg.

Le tableau 6 indique les activités vis-à-vis des granulomes au pellet de coton. Deux pellets de coton de 70 mg sont implantés sous la peau du dos de rats mâles Sprague-Dawley anesthésiés à l'éther. A partir du lendemain le produit est administré par voie orale tous les jours pendant 10 jours. Les rats sont sacrifiés et les pellets prélevés puis pesés avant et après séchage à 50 °C pendant 24 heures. On prend en considération les poids obtenus après déduction des poids des implants. Les moyennes des quantités ainsi calculées sont déterminées par lot et comparées aux moyennes du lot témoin.

Tableau 6

| Produit de l'exemple | dose en mg/kg | % d'inhibition pellets humides | vis à vis des pellets secs |
|---|---|---|---|
| 2 | 150 | 12 | 5 |
| | 300 | 17 | 23 |
| 3 | 150 | 0 | 5 |
| 4 | 150 | 13 | 10 |
| | 300 | 14 | 19 |
| Acide niflumique | 50 | 3 | 12 |

L'activité ulcérigène est déterminée chez le rat femelle Wistar d'après une variante de la technique décrite par J. M. Lwoff J. Pharmacol., 1971, 2, 1, 81-3. La substance à éprouver est administrée quotidiennement pendant 3 jours à des animaux mis à la diète hydrique. Les estomacs sont prélevés après 1, 2 et 3 jours de traitement et les ulcérations côtées de 0 à 4. L'indice d'ulcération est calculé pour chaque lot d'animaux comme étant le produit (moyenne des cotations × pourcentage d'animaux atteints) en distinguant les ulcérations aiguës des ulcérations en voie de cicatrisation. Les résultats sont représentés dans le tableau 7.

Tableau 7

| Traitement | dose administrée en mg/kg | Indice d'ulcération | | | | | |
|---|---|---|---|---|---|---|---|
| | | aiguë | | | en voie de cicatrisation | | |
| | | Jour 1 | Jour 2 | Jour 3 | Jour 1 | Jour 2 | Jour 3 |
| Témoins | | 0 | 1 | 36 | 0 | 0 | 2 |
| Produit exemple 2 | 150 | 6 | 2 | 48 | 0 | 6 | 28 |
| Produit exemple 3 | 150 | 6 | 20 | 33 | 0 | 6 | 12 |
| Produit exemple 4 | 150 | 0 | 0 | 72 | 0 | 8 | 16 |
| Acide niflumique | 100 | 330 | 1 | 119 | 0 | 400 | 378 |

L'action antidiabétique des produits de la présente invention a été démontrée de la façon suivante.

Des rats femelles de 180-200 g reçoivent par voie intrapéritonéale au jour J0 une dose de 100 mg/kg de streptozotocine en tampon citrate 0,05 M à pH 4,5 à raison de 0,5 ml pour 100 g de poids corporel. Au jour J2 les animaux hyperglycémiques sont sélectionnés par détermination de la glycémie par la méthode à l'orthotoluidine en flux continu après prélèvement au sinus rétro orbitaire. Seuls sont retenus les animaux ayant une glycémie supérieure ou égale à 2,5 g/l. Au jour J3, sans mise à jeun préalable les animaux sélectionnés reçoivent le traitement. Celui-ci consiste en une administration, par voie orale, du produit à étudier dans une suspension de gomme arabique à 6 % dans de l'eau sous un volume de 0,5 ml pour 100 g de poids corporel.

Les animaux sont sacrifiés une, deux ou quatre heures après le traitement et leur glycémie est déterminée. Chaque lot d'animaux traités est comparé à un lot d'animaux témoins recevant la suspension de gomme arabique seule. La substance prise comme référence est la metformine.

Les résultats sont donnés dans le tableau 8 qui indique le pourcentage de diminution de la glycémie sous l'influence du traitement. La mention NS signifie que la différence entre la glycémie des animaux traités et des animaux témoins est non significative, la mention S signifiant que cette différence est significative d'après le test t de Student à $p \leq 0,05$.

(Voir Tableau 8, p. 9)

Tableau 8

| PRODUIT | POURCENTAGE DE DIMINUTION de la glycémie en fonction de la dose administrée exprimée en mg/kg | | | |
|---|---|---|---|---|
| | 50 | 100 | 200 | 400 |
| N-(méthyl-5 thiénylidène-2) amino-5 benzodioxole-1,3 | | | | |
| traitement 1 h avant le sacrifice | 13 S | 22 S | 24 S | |
| 2 h avant le sacrifice | 22 S | 32 S | 35 S | |
| 4 h avant le sacrifice | 12 S | 27 S | 31 S | |
| N-(pyridylidène-3) amino-5 benzodioxole-1,3 | | | | |
| traitement 1 h avant le sacrifice | 16 S | NS | NS | |
| 2 h avant le sacrifice | NS | 27 S | 19 S | |
| 4 h avant le sacrifice | 23 S | 29 S | 28 S | |
| Metformine | | | | |
| traitement 1 h avant le sacrifice | | | NS | 14 S |
| 2 h avant le sacrifice | | | NS | NS |
| 4 h avant le sacrifice | | | NS | 15 S |

Compte tenu de leurs propriétés pharmacologiques, les produits faisant l'objet de la présente invention sont utiles en thérapeutique dans le traitement des affections à composantes algique et inflammatoire. A titre d'exemple ces produits peuvent être prescrits en rhumatologie (par exemple, rhumatismes inflammatoires et dégénératifs, atteintes articulaires et abarticulaires, crises aiguës de goutte), en traumatologie (par exemple, entorses, fractures, luxations, tendinites), en chirurgie (douleurs pré- et post-opératoires, en rééducation fonctionnelle, en dermatophlébologie (phlébites, périphlébites, varices, ulcérations cutanées), ainsi que dans les pathologies douloureuses et inflammatoires dentaires, ORL, cancéreuses, viscérales, vasculaires, neurologiques, etc.

Les produits de la présente invention sont également utiles en thérapeutique, en complément du régime alimentaire, dans le traitement du diabète non-insulino-dépendant. Ils pourront être employés, dans certains types de diabètes insulinodépendants, associés aux sulfamides hypoglycémiants ou à l'insulinothérapie.

Ces produits peuvent être administrés, en association avec les excipients usuels, par les voies orale, rectale, percutanée sous de nombreuses formes galéniques telles que comprimés, gélules, suppositoires, pommades, gels.

Les formes orales contiennent 100 à 500 mg de principe actif par forme unitaire, les formes rectales 100 mg à 1 g et les formes topiques 2 à 10 %. La posologie moyenne quotidienne est de 200 mg à 2 g de principe actif pour les voies orale et rectale.

## Revendications

1. Nouveaux produits de formule générale

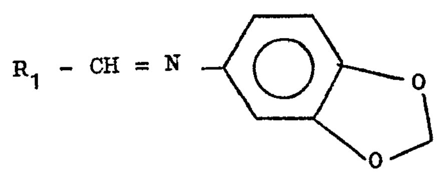

avec $R_1$ = reste hétérocyclique choisi dans le groupe

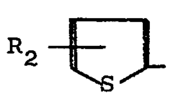 , 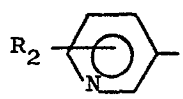 , 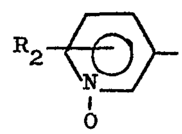 , 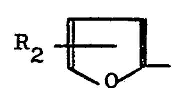

et $R_2$ = H ou un ou plusieurs substituants choisis dans le groupe $CH_3$, halogène, nitro.

2. Nouveaux produits selon la revendication 1 de formule générale

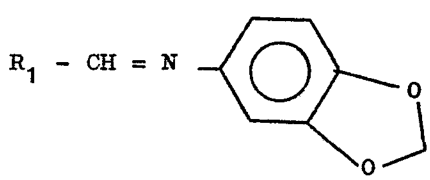

avec $R_1$ = 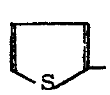 , 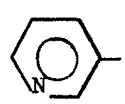 , 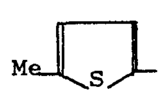 ou 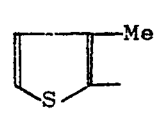

3. Procédé de préparation des produits selon les revendications 1 et 2 caractérisé en ce que l'on fait réagir l'aldéhyde $R_1CHO$ ($R_1$ ayant la signification donnée à la revendication 1) avec l'amine

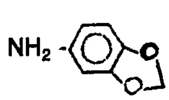

dans un solvant tel que le benzène et en ce que l'on élimine l'eau au fur et à mesure de sa formation.

4. Nouveaux médicaments utiles en thérapeutique contenant comme principe actif au moins un des produits selon les revendications 1 et 2.

**Claims**

1. New products with a general formula of

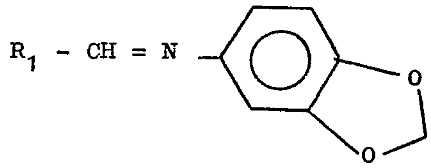

where $R_1$ = the heterocyclical remained chosen from the group

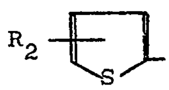 , 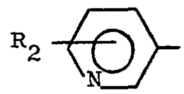 , 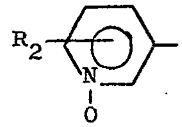 , 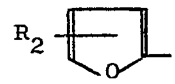

and $R_2$ = H or one or several subsitutes chosen from the group $CH_3$, hologen, nitro.

2. New products in accordance with Claim 1 with a general formula of

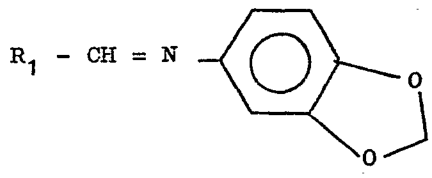

with $R_1$ = 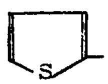 , 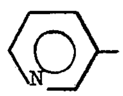 , 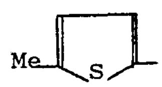 or 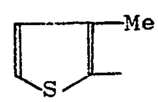

3. Process for the preparation of products according to the Claims 1 and 2 and characterised by the fact that the Aldehyde $R_1CHO$ ($R_1$ having the meaning given in Claim 1) is made to react with the amin :

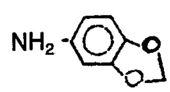

in a solvent such as benzene, and by the fact that the water is eliminated as and when it is formed.

4. New medecines useful in therapy containg as main active agent at least one of the products according to Claims 1 and 2.

**Ansprüche**

1. Neue Produkte allgemeiner Formel

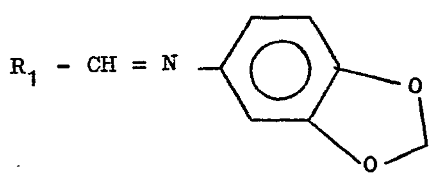

mit $R_1$ = heterozyklisches, in der Gruppe

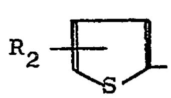 , 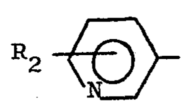 , 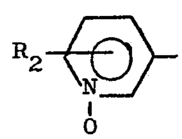 , 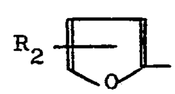

ausgewähltes Rest = H oder ein oder mehrere in der Gruppe $CH_3$, Hologen, nitro, ausgewählten Substituenden.

2. Neue Produkte nach Anspruche 1 allgemeiner Formel

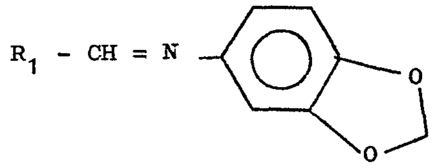

mit $R_1$ = 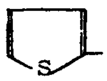, 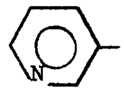, 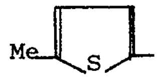 oder 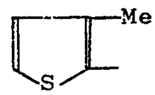

3. Produktevorbereitungsverfahren nach Ansprüchen 1 und 2 dadurch gekennzeichnet, dass der Aldehyd $R_1CHO$ ($R_1$ hat die im Anspruch 1 gegebene Bedeutung) mit dem Amin

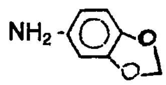

in einem Lösungsmittel, wie z.B. Benzen, zur Reaktion gebracht wird und dass Wasser je nach seiner Bildung entfernt wird.

4. Neue, in der Therapie nützliche Medikamente, die mindestens ein Produkt gemäss Ansprüche 1 und 2 als aktiven Stoff enthalten.